(19) **Europäisches Patentamt / European Patent Office / Office européen des brevets**

(11) **EP 1 597 171 B1**

(12) **EUROPEAN PATENT SPECIFICATION**

(45) Date of publication and mention
of the grant of the patent:
**19.12.2012 Bulletin 2012/51**

(51) Int Cl.:
$A61K\ 31/08$ (2006.01)    $A61K\ 33/00$ (2006.01)
$A61K\ 45/06$ (2006.01)    $A61K\ 9/00$ (2006.01)
$A61K\ 9/12$ (2006.01)

(21) Application number: **04700472.6**

(22) Date of filing: **07.01.2004**

(86) International application number:
**PCT/GB2004/000026**

(87) International publication number:
**WO 2004/062461 (29.07.2004 Gazette 2004/31)**

(54) **THERAPEUTIC MICROFOAM**

THERAPEUTISCHER MIKROSCHAUM

MICRO-MOUSSE THERAPEUTIQUE

(84) Designated Contracting States:
**AT BE BG CH CY CZ DE DK EE ES FI FR GB GR HU IE IT LI LU MC NL PT RO SE SI SK TR**

(30) Priority: **10.01.2003 GB 0300586**

(43) Date of publication of application:
**23.11.2005 Bulletin 2005/47**

(73) Proprietor: **BTG International Limited**
**London EC4M 7RD (GB)**

(72) Inventors:
• **HARMAN, Anthony, David**
**Henley-on-Thames,**
**Oxon RG9 5JP (GB)**
• **WRIGHT, David, Dakin, Iorwerth**
**High Wycombe HP11 1PT (GB)**

(74) Representative: **England, Christopher David et al**
**BTG International Limited**
**5 Fleet Place**
**London EC4M 7RD (GB)**

(56) References cited:
**WO-A-02/41872**      **WO-A-03/099681**
**US-A1- 2002 077 589**

• **DATABASE MEDLINE [Online] US NATIONAL LIBRARY OF MEDICINE (NLM), BETHESDA, MD, US; September 1997 (1997-09), KIRSCH L E ET AL: "Pharmaceutical container/closure integrity. I: Mass spectrometry-based helium leak rate detection for rubber-stopped glass vials." XP002289788 Database accession no. NLM9357304 & PDA JOURNAL OF PHARMACEUTICAL SCIENCE AND TECHNOLOGY / PDA. 1997 SEP-OCT, vol. 51, no. 5, September 1997 (1997-09), pages 187-194, ISSN: 1079-7440**

**Description**

The present invention relates to a therapeutic microfoam comprising a sclerosing material, particularly a sclerosing liquid, which is suitable for use in the treatment of various medical conditions involving blood vessels, particularly varicose veins and other disorders involving venous malformation. The invention relates also to the method and apparatus for the generation of such a microfoam.

Sclerosis of varicose veins is based on the injection into the veins of liquid sclerosant substances which, by *inter alia* causing a localised inflammatory reaction, favour the elimination of these abnormal veins. Until recently, sclerotherapy was a technique selected in cases of small and medium calibre varicose veins, those with diameters equal to or greater than 7 mm being treated by surgery.

An injectable microfoam suitable for therapeutic use, on larger veins in particular, has now been developed and is described in EP-A-0656203 and US 5676962. These patents describe a low-density microfoam produced with a sclerosing substance which, when injected into a vein, displaces blood and ensures that the sclerosing agent contacts the endothelium of the vessel in a known concentration and for a controllable time, achieving sclerosis of the entire segment occupied.

The preparation of such a microfoam may be carried out with a solution of any sclerosing substance, particularly polidocanol. The method of preparation is to use a small brush attached to a high-speed motor to whip a dilute aqueous solution of the preferred sclerosant to a firm mousse-like consistency in a period of 1-2 minutes under a gas atmosphere containing physiologically acceptable gas mixes. However, this known method requires extemporaneous production of microfoam by the physician, pharmacist or an assistant immediately prior to administration to the patient. Such procedure allows for variation of microfoam sclerosing agent depending upon the person preparing it; microfoam density, gas makeup, bubble size and foam stability all needing attention with respect to the condition being treated.

A solution to this problem is offered in WO 00/72821-A1 (BTG International Limited), which provides a method and a number of different devices that are capable of producing a uniform injectable microfoam. This microfoam is made with a relatively low concentration of a foamable sclerosing agent and a significant amount of a blood dispersible gas in sterile fashion without volatile liquid propellants or the need for the operator to directly be concerned in the control of its parameters. This application also addresses the perception that large volumes of nitrogen should not unnecessarily be introduced into patients. This is particularly an issue where large vessels are being filled with foam, if air is used as the gas for producing the foam. A preferred form of gas described in WO 00/72821-A1 comprises 50% vol/vol or more oxygen, the remainder being carbon dioxide, or carbon dioxide, nitrogen and trace gases in the proportion found in atmospheric air. Preferably the sclerosing agent is a solution of polidocanol or sodium tetradecyl sulfate in an aqueous carrier, e.g. water, particularly in a saline.

Various issues with long-term storage are not addressed in WO 00/72821-A1. One of these is a potential problem with storing the sclerosing fluid, for example, aqueous polidocanol, in the presence of oxygen. WO 02/41872-A1 (BTG International Limited), offers a solution to this potential problem by storing the sclerosant liquid and the oxygen-rich physiologically acceptable blood dispersible gas in separate containers until immediately prior to use, when the blood-dispersible gas is introduced into the container holding the sclerosant liquid. The mixture of blood-dispersible gas and sclerosant liquid is then released, the components of the mixture interacting upon release of the mixture to form a sclerosing foam.

Kirsch et al (1997, J Pharm Sci Tech, 51 (5), 187-194) discloses to the use of helium as tracer to test integrity of rubber stoppered vials having test pinholes engineered therein.

The present inventors have identified another issue with long-term storage of physiologically acceptable blood dispersible gases under pressure in a sealed canister, namely the need to ensure that potential leaks are minimised. They have determined that the introduction of helium into a physiologically acceptable blood dispersible gas gives a similarly physiologically acceptable mixture that is capable of being detected at very small quantities by a suitable sensor.

Although helium has very low solubility in water or blood, the helium gas molecules are small enough to readily diffuse across pulmonary gas exchange membranes and be exhaled. The safety of helium in respirable gas mixtures is well established and widely exploited (namely Heliox mixtures for deep sea divers containing up to 70% helium).

The advantage of helium in respirable gas mixtures results from its extremely low solubility in water or blood, even under high ambient pressures. Helium can also be shown to diffuse very rapidly across pulmonary gas exchange membranes, and therefore presents no danger of pulmonary gas embolism. Helium can also be used as an efficient marker of gas bubble arrival in the pulmonary circulation, following breakdown of a microfoam that has helium as a constituent gas.

In its broadest sense the present invention relates to subject-matter as defined in the appended claims.

Accordingly the first aspect of the present invention provides a sclerosing foam comprising a physiologically acceptable gas that is readily dispersible in blood together with an aqueous sclerosant liquid, characterised in that the gas comprises 75% vol/vol or more oxygen and/or carbon dioxide and less than 10% vol/vol nitrogen and the foam is a microfoam further including helium in an amount from 0.5% to 20% vol/vol of the total volume of gas.

A commercially available leak detector (or "sniffer") is the Veeco™ MS-40 portable automatic leak detector, provided by the Vacuum Instrument Corporation, Ronkonkoma, New York. This is said to detect a helium leakage level expressed in units of std cc/sec down to $4 \times 10^{-11}$, i.e. $4 \times 10^{-11}$ $cm^3$ $s^{-1}$ at standard temperature conditions.

In a typical device of the type disclosed in WO 00/72821-A1, a pressure loss of 0.15 bar in 3 years shelf life may be tolerated from a pressurised single-canister microfoam generator of 300 ml capacity, initially at 3.5 bar absolute, and containing 18 ml of a sclerosing liquid. Therefore the volume of gas lost from the canister in 3 years is given by $V$, where:

$$V = \frac{0.15}{1.00} \times (300 - 18) = 42.3 \text{ cm}^3$$

This loss of 42.3 $cm^3$ gas in 3 years corresponds to an average leak rate of :

$$\frac{42.3}{60 \times 60 \times 24 \times 365 \times 3} = 1.34 \times 10^{-6} \text{ cm}^3 \text{ s}^{-1}$$

Thus, if 3% helium were to be incorporated in the gas mixture, a leakage level of 3% of this figure, namely $4 \times 10^{-8}$ $cm^3$ $s^{-1}$, would have to be detected. This is well within the ability of commercially available leak detectors such as the $V_{eeco}$™ MS-40 portable automatic leak detector.

The limits of the present invention are a sclerosing foams including helium in an amount from 0,5% to 20% VOL/VOL of the total volume of gas. Similar calculations to the above show that the leakage level that has to be detected is $1 \times 10^{-10}$ $cm^3$ $s^{-1}$ to $5 \times 10^{-6}$ $cm^3$ $s^{-1}$, again within the ability of commercially available leak detectors.

The microfoam includes helium in an amount from 0.5% to 20% of the total volume of gas. More preferably the microfoam includes helium in an amount from 1% to 10% of the total volume of gas. More preferably the microfoam includes helium in an amount from 1% to 5% of the total volume of gas.

The gas mixture may be regarded as made up of three components:

- the physiologically acceptable gas or gases;
- helium; and optionally
- a further inert gas or gases.

Suitable further inert gases include neon, argon, and nitrogen. The gas mixture includes less than 10% vol/vol nitrogen.

The gas mixture comprises 75% or more oxygen and/or carbon dioxide and most preferably at least 99% oxygen or carbon dioxide. Preferably the oxygen or carbon dioxide is medical grade.

In a second aspect of the present invention there is provided a method for producing a microfoam suitable for use in scleropathy of blood vessels, comprising introducing a physiologically acceptable blood-dispersible gas into a container holding an aqueous sclerosant liquid and releasing the mixture of blood-dispersible gas and sclerosant liquid, whereby upon release of the mixture the components of the mixture interact to form a microfoam, characterised in that the physiologically acceptable blood-dispersible gas comprises 75% vol/vol or more oxygen and/or carbon dioxide and less than 10% vol/vol nitrogen which is stored in the presence of helium in an amount from 0.5% to 20% vol/vol of the total volume of gas. The pressurised gas mixture may be stored long term in the same container as the aqueous sclerosant liquid, if long term stability tests show no degradative reaction between the gas mixture and the aqueous sclerosant liquid.

Alternatively the oxygen component of the final gas mix is stored in a separate container from the aqueous sclerosant liquid and introduced immediately prior to use. The oxygen component of the gas may thereby be stored in a container provided with engaging means for the container holding the aqueous sclerosant liquid. Such an engaging means is disclosed in WO 02/41872-A1

In a third aspect of the present invention there is provided a device for producing a microfoam suitable for use in scleropathy of blood vessels, comprising a housing in which is situated a pressurisable chamber containing a solution of the sclerosing agent in a physiologically acceptable solvent; a pathway with one or more outlet orifices by which the solution may pass from the pressurisable chamber to the exterior of the device through said one or more outlet orifices and a mechanism by which the pathway from the chamber to the exterior can be opened or closed such that, when the container is pressurised and the pathway is open, fluid will be forced along the pathway and through the one or more outlet orifices;

said housing incorporating an inlet for the admission of a pressurised source of physiologically acceptable gas that is dispersible in blood; the gas being in contact with the solution on activation of the mechanism such as to produce a gas-solution mixture;

said pathway to the exterior of the housing including one or more foaming elements;
characterised in that the housing is charged with blood-dispersible gas comprising 75% vol/vol or more oxygen and/or carbon dioxide and less than 10% vol/vol nitrogen which is stored in the presence of helium in an amount from 0.5% to 20% vol/vol of the total volume of gas.

The present disclosure provides a method of treating a patient in need of sclerotherapy of a blood vessel comprising administering a microfoam as described above. There is further provided the use of such a microfoam in the manufacture of a medicament for sclerotherapy.

The suitability of using helium in foam sclerotherapy techniques has already been determined by J. García Mingo. See his contribution to "Foam Sclerotherapy: State of the Art" (Editions Phlébologiques Françaises), edited by Jean-Paul Henriet, pages 45-50.

The sclerosant liquid utilised in the invention may be any of those discussed in WO 00/72821-A1 and WO 02/41872-A1. Preferably the sclerosant liquid is a solution of polidocanol or sodium tetradecyl sulfate in an aqueous carrier, e.g. water, particularly in a saline. More preferably the solution is from 0.25 to 5% vol/vol polidocanol, preferably in sterile water or a physiologically acceptable saline, e.g. in 0.5 to 2% vol/vol saline. More preferably still, the concentration of polidocanol is from 0.5 to 5% vol/vol in the liquid, preferably 0.5 to 3% vol/vol polidocanol and most preferably being 1% vol/vol in the liquid. Concentration of sclerosant in the solution will be advantageously increased for certain abnormalities such as Klippel-Trenaunay syndrome.

The sclerosant may also contain additional components, such as stabilising agents, e.g. foam stabilising agents, e.g. such as glycerol. Further components may include alcohols such as ethanol. Even though this can reduce foam stability, inclusion of a few percent of ethanol is thought to aid in solubilising low-molecular-weight oligomers of polidocanol and also prevent degradation of the polidocanol.

The water or saline also may contain 2-5% vol/vol physiologically acceptable alcohol, e.g. ethanol. The polidocanol solution is preferably phosphate buffered.

Addition of glycerol to the aforesaid sclerosant imparts a longer half-life to the resultant foam.

For the purpose of this application terms have the following definitions. Physiologically acceptable blood dispersible gas is a gas that is capable of being substantially completely dissolved in or absorbed by blood. A sclerosant liquid is a liquid that is capable of sclerosing blood vessels when injected into the vessel lumen. Scleropathy or sclerotherapy relates to the treatment of blood vessels by injection of a sclerosing agent to eliminate them. An aerosol is a dispersion of liquid in gas. Half-life of a microfoam is the time taken for half the liquid in the microfoam to revert to unfoamed liquid phase, under the influence of gravity, and at a defined temperature.

The mixture of blood-dispersible gas and sclerosant liquid is preferably pressurised to a pre-determined level. Preferred pressures are in the range 800 mbar to 4.5 bar gauge (1.8 bar to 5.5 bar absolute). Pressures in the range of 1 bar to 2.5 bar gauge have been found to be particularly effective-over this range of pressures, there is very little change in either the density or the half-life of the resulting foam as the canister empties.

Preferably the microfoam is such that less than 20% of the bubbles are less than 30 $\mu$m diameter, greater than 75% are between 30 and 280 $\mu$m diameter, less than 5 % are between 281 and 500 $\mu$m diameter, and there are substantially no bubbles greater than 500 $\mu$m diameter.

Preferably the gas/liquid ratio in the mix is controlled such that the density of the microfoam is 0.07 g/ml to 0.19 g/ml, more preferably 0.10 g/ml to 0.15 g/ml.

Preferably the microfoam has a half-life of at least 2 minutes, more preferably at least 2.5 minutes. The half-life may be as high as 1 or 2 hours or more, but is preferably less than 60 minutes, more preferably less than 15 minutes and most preferably less than 10 minutes.

The present invention will now be described further by way of illustration only by reference to the following Figures and Examples. Further embodiments falling within the scope of the invention will occur to those skilled in the art in the light of these.

FIGURES

Figure 1 shows a cross-sectional view of a pre-pressurised container for the generation of therapeutic microfoam according to the invention, as disclosed in WO 00/72821-A1 and further described in Example 1 below.

Figure 2 shows a shows a cross-sectional view of a device comprising a container provided with engaging means and a mesh stack shuttle according to the invention, as disclosed in WO 02/41872-A1 and further described in Example 2 below.

Figure 3 shows an apparatus for use in then helium detection technique as further described in Example 3 below.

## EXAMPLES

### Example 1-pre-pressurised container

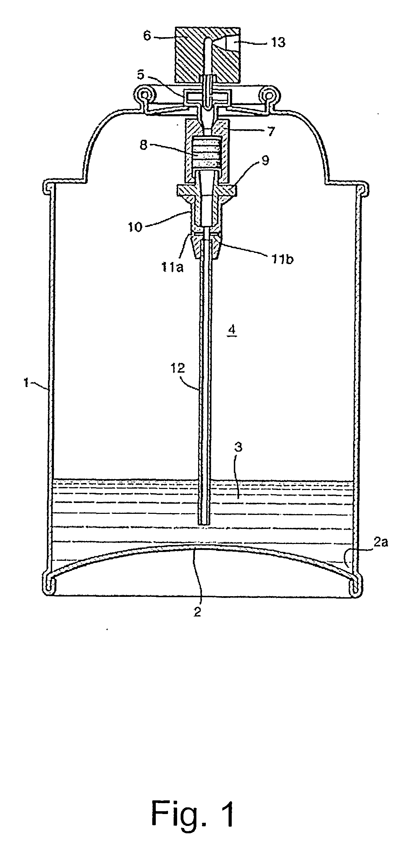

A typical apparatus for the generation of therapeutic microfoam according to the invention, as disclosed in WO 00/72821-A1, is shown in Figure 1.

The canister has an aluminium wall (1), the inside surface of which is coated with an epoxy resin. The bottom of the canister (2) is domed inward. The canister inner chamber (4) is pre-purged with 100% oxygen for 1 minute, containing 15 ml of a 1% vol/vol polidocanol /20 mmol phosphate buffered saline solution / 4% ethanol, of composition as given in Table 1 below, then filled with an oxygen-helium mixture at 2.7 bar gauge (1.7 bar over atmospheric). This is provided by introducing a charge of helium and then overpressuring the polidocanol part filled can with 1.7 bar oxygen.

A typical gas mixtures is 3% He, 25 and 35% $CO_2$, with the balance $O_2$ as a final gas mixture at approx 3.5 bar absolute.

A standard 1 inch diameter Ecosol™ aerosol valve (5) (Precision Valve, Peterborough, UK) is crimped into the top of the canister after sterile part filling with the solution and may be activated by depressing an actuator cap (6) to release content via an outlet nozzle (13) sized to engage a Luer fitting of a syringe or multi-way connector (not shown). A further connector (7) locates on the bottom of the standard valve and mounts four Nylon 66 meshes held in high density polyethylene (HDPE) rings (8), all within an open-ended polypropylene casing. These meshes have diameter of 6 mm and have a 14% open area made up of 20 $\mu$m pores, with the meshes spaced 3.5 mm apart.

A further connector (9) locates on the bottom of the connector holding the meshes and receives a housing (10) which mounts the dip tube (12) and includes gas receiving holes (11a, 11b) which admit gas from chamber (4) into the flow of liquid which rises up the dip-tube on operation of the actuator (6). These are conveniently defined by an Ecosol™ device provided by Precision Valve, Peterborough, UK, provided with an insert. Holes (11 a, 11b) have cross-sectional area such that the sum total ratio of this to the cross-sectional area of the liquid control orifice at the base of the valve housing (at the top of the dip-tube) is controlled to provide the required gas/liquid ratio.

### Example 2-container with engaging means and mesh stack shuttle

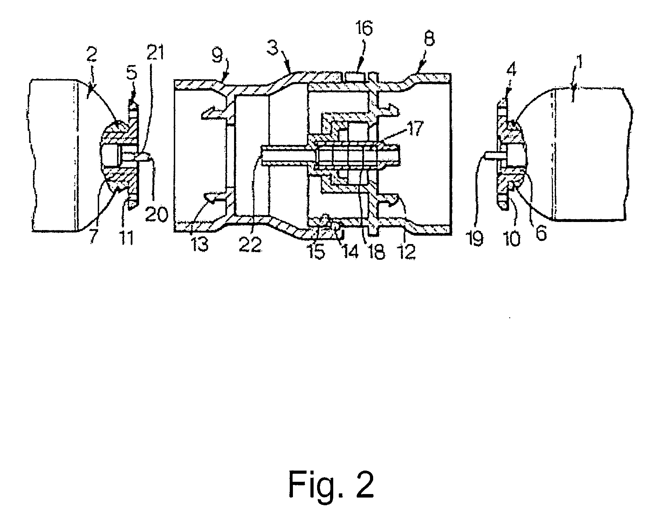

A device comprising a container provided with engaging means and a mesh stack shuttle according to the invention, as disclosed in WO 02/41872-A1, is shown in Figure 2. The device comprises a low pressure container (1) for an aqueous sclerosant liquid and an unreactive gas atmosphere, a container (2) for a physiologically acceptable blood-dispersible gas and an engaging means comprising a connector (3).

The container (2) for a physiologically acceptable blood-dispersible gas is charged at 5.8 bar absolute pressure with an oxygen-helium mixture containing 3% helium, whereas the container (1) is charged with a carbon dioxide-helium mixture containing 3% helium. Container (2) is used to pressurise container (1) at the point of use to approx 3.5 bar absolute and is then discarded, just before the microfoam is required. The two containers will thus be referred to hereinafter as the PD [polidocanol] can (1) and the $O_2$ can (2).

Each of the cans (1, 2) is provided with a snap-fit mounting (4, 5). These may be made as identical mouldings. The snap-fit parts (4, 5) engage the crimped-on mounting cup (6, 7) of each can (1, 2) with high frictional force. The connector is made in two halves (8, 9), and the high frictional force allows the user to grip the two connected cans (1, 2) and rotate the connector halves (8, 9) relative to each other without slippage between connector (3) and cans. Each of these can mountings (6, 7) has snap-fit holes (10, 11) for engaging mating prongs (12, 13) which are on the appropriate surfaces of the two halves (8, 9) of the connector.

The connector (3) is an assembly comprising a number of injection mouldings. The two halves (8, 9) of the connector are in the form of cam track sleeves which fit together as two concentric tubes. These tubes are linked by proud pins (14) on one half that engage sunken cam tracks (15) on the other half. The cam tracks have three detented stop positions. The first of these detents is the stop position for storage. An extra security on this detent is given by placing a removable collar (16) in a gap between the end of one sleeve and the other. Until this collar (16) is removed it is not possible to rotate the sleeves past the first detent position. This ensures against accidental actuation of the connector.

The cam track sleeves (8, 9) are injection moulded from ABS as separate items, and are later assembled so that they engage one another on the first stop of the detented cam track. The assembled sleeves are snap-fitted as a unit onto the $O_2$ can (2) mounting plate (5) via four locating prongs. The security collar is added at this point to make an $O_2$ can subassembly.

The connector (3) includes in its interior a series of foaming elements comprising a mesh stack shuttle (17) on the connector half (8) adjacent to the PD can (1). The mesh stack shuttle (17) is comprised of four injection moulded disk filters with mesh hole size of 20 $\mu$m and an open area of approx. 14%, and two end fittings, suitable for leak-free connection to the two canisters. These elements are preassembled and used as an insert in a further injection moulding operation that encases them in an overmoulding (18) that provides a gas-tight seal around the meshes, and defines the outer surfaces of the mesh stack shuttle. The end fittings of the stack (17) are designed to give gas-tight face and/or rim seals against the stem valves (19, 20) of the two cans (1, 2) to ensure sterility of gas transfer between the two cans.

The mesh stack shuttle (17) is assembled onto the PD can valve (19) by push-fitting the components together in a aseptic environment.

The PD can (1) and attached shuttle (17) are offered up to the connector (3) and the attached $O_2$ can (2), and a sliding fit made to allow snap-fitting of the four locating prongs (12) on the PD can side of the connector (3) into the mating holes (10) in the mounting plate (4) on the PD can (1). This completes the assembly of the system. In this state, there is around 2 mm of clearance between the stem valve (20) of the $O_2$ can (2) and the point at which it will form a seal against a female Luer outlet from the stack.

When the security collar (16) is removed, it is possible to grasp the two cans (1, 2) and rotate one half of the connector (3) against the other half to engage and open the $O_2$ can valve (20).

As the rotation of the connector (3) continues to its second detent position, the PD can valve (19) opens fully. The gas flow from the $O_2$ can (2) is restricted by a small outlet hole (21) in the stem valve (20). It takes about 45 seconds at the second detent position for the gas pressure to (almost) equilibrate between the two cans to a level of 3.45 bar $\pm$ 0.15 bar.

After the 45 second wait at the second detent position, the connector (3) is rotated further to the third detent position by the user. At this position, the two cans (1, 2) can be separated, leaving the PD can (1) with half (8) of the connector and the shuttle assembly (17) captive between the connector and the PD can. The $O_2$ can (2) is discarded at this point.

A standard 1 inch diameter aerosol valve (19) (Precision Valve, Peterborough, UK) is crimped into the top of the PD can (1) before or after sterile filling with the solution and may be activated by depressing the mesh stack shuttle (17), which functions as an aerosol valve actuator mechanism, to release the contents via an outlet nozzle (22) sized to engage a Luer fitting of a syringe or multi-way connector (not shown).

**Example 3-helium detection technique**

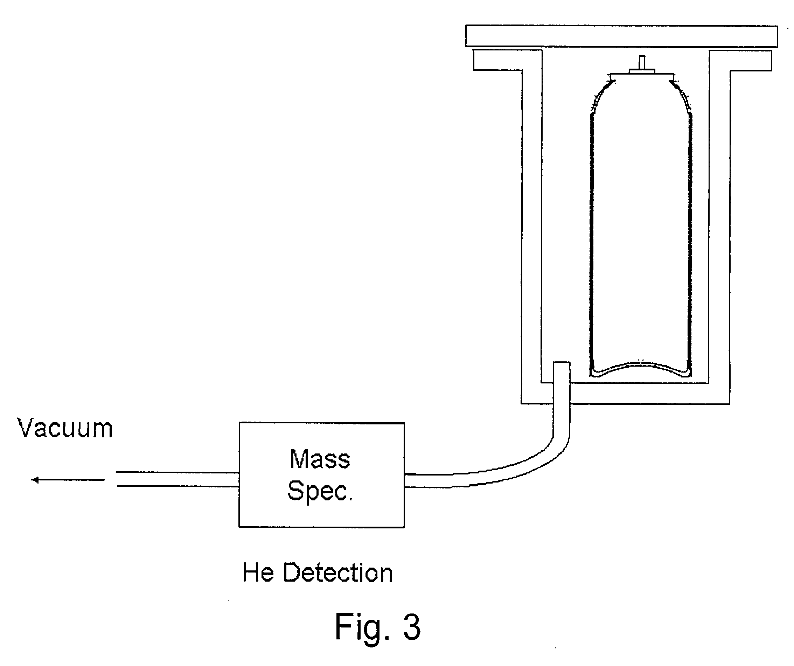

A leak detector incorporating an apparatus for the generation of therapeutic microfoam according to the invention is shown in Figure 3. The device uses a commercially available leak detector, the Veeco™ MS-40 portable automatic leak detector, provided by the Vacuum Instrument Corporation, Ronkonkoma, New York.

The leak detector uses a large capacity internal mechanical pump and a mass spectrometer comprising a 180-degree deflection dual magnetic sector mass spectrometer tube with built-in high vacuum ion gauge. The mass spectrometer is sensitive to Helium Mass 3 or Mass 4 and is operator selectable.

An apparatus for the generation of therapeutic microfoam according to the invention, such as described in Examples 1 or 2, is placed is a sealed chamber. The space between the generator and the sealed chamber is then evacuated using the internal mechanical pump, and helium levels, emanating from the generator into the sealed, evacuated space around it, are detected using the mass spectrometer.

**Table 1-Composition of 1% Polidocanol solution**

| Material | Quantities | |
|---|---|---|
| | **% w/w** | **per 1000 g** |
| Polidocanol | 1.000 | 10.00 g |
| Ethanol 96% EP | 4.200 | 42.00 g |
| Disodium Hydrogen Phosphate Dihydrate. EP | 0.240 | 2.40 g |
| Potassium Di-hydrogen Phosphate. EP | 0.085 | 0.85 g |
| 0.1 M Sodium Hydroxide Solution [used for adjustment of pH: 7.2-7.5] | q.s. | q.s. |
| 0.1 M Hydrochloric Acid | q.s. | q.s. |
| Water for injection. EP [used to adjust to final weight] | approx. 94.475 q.s. to 100.00% | approx. 944.75 g q.s. to 1000.00 g |
| **TOTAL:** | 100.00% | 1000.00 g |

**Claims**

1. A sclerosing foam comprising a physiologically acceptable gas that is readily dispersible in blood together with an aqueous sclerosant liquid, **characterised in that** said gas comprises 75% vol/vol or more oxygen and/or carbon dioxide and less than 10% vol/vol nitrogen and the foam is a microfoam further including helium in an amount from 0.5% to 20% vol/vol of the total volume of gas.

2. A sclerosing foam as claimed in claim 1, **characterised in that** the microfoam includes helium in an amount from 1% to 10% of the total volume of gas.

3. A sclerosing foam as claimed in claim 2, **characterised in that** the microfoam includes helium in an amount from 1% to 5% of the total volume of gas.

4. A sclerosing foam as claimed in claim 3, **characterised in that** the gas mixture comprises at least 99% oxygen or carbon dioxide.

5. A sclerosing foam as claimed in any preceding claim, **characterised in that** the sclerosant liquid is a solution of polidocanol or sodium tetradecyl sulfate in an aqueous carver.

6. A sclerosing foam as claimed claim 5, **characterised in that** the sclerosant liquid is a solution of 0.25 to 5% vol/vol polidocanol.

7. A sclerosing foam as claimed in any preceding claim, **characterised in that** the microfoam is such that less than 20% of the bubbles comprising the microfoam are less than 30 $\mu$m diameter, greater than 75% are between 30 and 280 $\mu$m diameter, less than 5 % are between 281 and 500 $\mu$m diameter, and there are substantially no bubbles greater than 500 $\mu$m diameter.

8. A sclerosing foam as claimed in any preceding claim, **characterised in that** the gas/liquid ratio in the mix is controlled such that the density of the microfoam is 0.07 g/ml to 0.19 g/ml.

9. A sclerosing foam as claimed in any preceding claim, **characterised in that** the microfoam has a half-life of at least 2 minutes.

10. A method for producing a microfoam suitable for use in scleropathy of blood vessels, comprising introducing a physiologically acceptable blood-dispersible gas into a container holding an aqueous sclerosant liquid and releasing the mixture of blood-dispersible gas and sclerosant liquid, whereby upon release of the mixture the components of the mixture interact to form a microfoam, **characterised in that** the physiologically acceptable blood-dispersible gas comprises 75% vol/vol or more oxygen and/or carbon dioxide and less than 10% vol/vol nitrogen which is stored in the presence of helium in an amount from 0.5% to 20% vol/vol of the total volume of gas.

11. A method as claimed in claim 10, **characterised in that** the oxygen component of the final gas mix is stored in a separate container from the aqueous sclerosant liquid and introduced immediately prior to use.

12. A device for producing a microfoam suitable for use in scleropathy of blood vessels, comprising a housing in which is situated a pressurisable chamber containing a solution of the sclerosing agent in a physiologically acceptable solvent; a pathway with one or more outlet orifices by which the solution may pass from the pressurisable chamber to the exterior of the device through said one or more outlet orifices and a mechanism by which the pathway from the chamber to the exterior can be opened or closed such that, when the container is pressurised and the pathway is open, fluid will be forced along the pathway and through the one or more outlet orifices;
said housing incorporating an inlet for the admission of a pressurised source of physiologically acceptable gas that is dispersible in blood; the gas being in contact with the solution on activation of the mechanism such as to produce a gas-solution mixture;
said pathway to the exterior of the housing including one or more foaming elements;
**characterised in that** the housing is charged with blood-dispersible gas comprising 75% vol/vol or more oxygen and/or carbon dioxide and less than 10% vol/vol nitrogen which is stored in the presence of helium in an amount from 0.5% to 20% vol/vol of the total volume of gas.

13. A sclerosing foam as claimed in any one of claims 1 to 10 for sclerotherapy.

**Patentansprüche**

1. Sklerosierender Schaum, umfassend ein physiologisch verträgliches Gas, das zusammen mit einer wässrigen Sklerosierungsmittelflüssigkeit leicht in Blut dispergierbar ist, **dadurch gekennzeichnet, dass** das Gas 75% Vol./Vol. oder mehr Sauerstoff und/oder Kohlendioxid und weniger als 10% Vol./Vol. Stickstoff umfasst und der Schaum ein Mikroschaum ist, der des Weiteren Helium in einer Menge von 0,5% bis 20% Vol./Vol. des gesamten Gasvolumens einschließt.

2. Sklerosierender Schaum wie in Anspruch 1 beansprucht, **dadurch gekennzeichnet, dass** der Mikroschaum Helium in einer Menge von 1% bis 10% des gesamten Gasvolumens einschließt.

3. Sklerosierender Schaum wie in Anspruch 2 beansprucht, **dadurch gekennzeichnet, dass** der Mikroschaum Helium in einer Menge von 1% bis 5% des gesamten Gasvolumens einschließt.

4. Sklerosierender Schaum wie in Anspruch 3 beansprucht, **dadurch gekennzeichnet, dass** die Gasmischung mindestens 99% Sauerstoff oder Kohlendioxid umfasst.

5. Sklerosierender Schaum wie in einem beliebigen der vorhergehenden Ansprüche beansprucht, **dadurch gekennzeichnet, dass** die Sklerosierungsmittelflüssigkeit eine Lösung von Polydocanol oder Natriumtetradecylsulfat in einem wässrigen Träger ist.

6. Sklerosierender Schaum wie in Anspruch 5 beansprucht, **dadurch gekennzeichnet, dass** die Sklerosierungsmittelflüssigkeit eine Lösung von 0,25 bis 5% Vol./Vol. Polydocanol ist.

7. Sklerosierender Schaum wie in einem beliebigen der vorhergehenden Ansprüche beansprucht, **dadurch gekennzeichnet, dass** der Mikroschaum so ist, dass weniger als 20% der Blasen die den Mikroschaum umfassen weniger als 30 μm Durchmesser aufweisen, mehr als 75% zwischen 30 und 280 μm Durchmesser aufweisen, weniger als 5% zwischen 281 und 500 μm Durchmesser aufweisen, und dass es im Wesentlichen keine Blasen gibt, die größer als 500 μm Durchmesser sind.

8. Sklerosierender Schaum wie in einem beliebigen der vorhergehenden Ansprüche beansprucht, **dadurch gekennzeichnet, dass** das Gas/Flüssigiceitsverhältnis in der Mischung so kontrolliert ist, dass die Dichte des Mikroschaums 0,07 g/ml bis 0,19 g/ml beträgt.

9. Sklerosierender Schaum wie in einem beliebigen der vorhergehenden Ansprüche beansprucht, **dadurch gekennzeichnet, dass** der Mikroschaum eine Halbwertszeit von mindestens 2 Minuten aufweist.

10. Verfahren zur Herstellung eins Mikroschaums, der geeignet ist zur Verwendung in Skleropathie von Blutgefäßen, umfassend Einrühren eines physiologisch verträglichen in Blut dispergierbaren Gases in einen Behälter, der eine wässrige Sklerosierungsmittelflüssigkeit beinhaltet und Freilassen der Mischung des in Blut dispergierbaren Gases und der Sklerosierungsmittelflüssigkeit, wobei bei der Freilassung der Mischung die Komponenten der Mischung interagieren, um einen Mikroschaum zu bilden, **dadurch gekennzeichnet, dass** das physiologische Blutdispergierbare Gas 75% Vol./Vol. oder mehr Sauerstoff und/oder Kohlendioxid und weniger als 10% Vol./Vol. Stickstoff welches in der Gegenwart von Helium in einer Menge von 0,5% bis 20% Vol./Vol. des gesamten Gasvolumens gelagert wird, umfasst.

11. Verfahren wie in Anspruch 10 beansprucht, **dadurch gekennzeichnet, dass** die Sauerstoffkomponente der finalen Gasmischung in einem von der wässrigen Sklerosierungsmittelfilüssigkeit getrennten Behälter gelagert wird und unmittelbar vor der Verwendung eingeführt wird.

12. Vorrichtung zur Herstellung eines Mikroschaums, geeignet zur Verwendung in der Skleropäthie von Blutgefäßen, umfassend ein Gehäuse in dem eine Kammer angeordnet ist, die unter Druck gesetzt werden kann, enthaltend eine Lösung des Sklerosierungsmittels in einem physiologisch verträglichen Lösungsmittel; einen Durchgang mit einer oder mehreren Auslassöffnungen, durch welche die Lösung aus der Kammer, welche unter Druck gesetzt werden kann, durch die eine oder mehrere Auslassöffnungen zu der Außenseite der Vorrichtung passieren kann, und einen Mechanismus, durch weichen der Durchgang aus der Kammer zu der Außenseite geöffnet oder geschlossen werden kann, so dass, wenn der Container unter Druck gesetzt wird und der Durchgang offen ist, das Fluid den Durchgang entlang und durch die eine oder mehreren Auslassöffnungen gezwungen wird, wobei das Gehäuse einen Einlass für die Aufnahme einer unter Druck gesetzten Quelle eines physiologisch verträglichen Gases, das in Blut dispergierbar ist, inkorporiert; wobei das Gas mit der Lösung bei der Aktivierung des Mechanismus in Kontakt ist, um ein Gas-Lösungsgemisch zu erzeugen; wobei der Durchgang zu der Außenseite des Gehäuses ein oder mehrere schaumbildende Elemente enthält; **dadurch gekennzeichnet, dass** das Gehäuse mit in Blut dispergierbarem Gas beladen ist, umfassend 75% Vol./Vol. oder mehr Sauerstoff und/oder Kohlendioxid und weniger als 10% Vol./Vol. Stickstoff, welches in der Gegenwart von Helium in einer Menge von 0,5 bis 20% Vol./Vol. des gesamten Gasvolumens gelagert ist.

**13.** Sklerosierender Schaum wie in einem beliebigen der Ansprüche 1 bis 10 beansprucht für die Sklerotherapie.

**Revendications**

**1.** Mousse sclérosante comprenant un gaz physiologiquement acceptable qui est aisément dispersible dans le sang conjointement avec un liquide sclérosant aqueux, **caractérisée en ce que** le gaz comprend 75 % vol/vol ou plus d'oxygène et/ou de dioxyde de carbone et moins de 10 % vol/vol d'azote et la mousse est une micromousse comprenant en outre de l'hélium en une quantité de 0,5 % à 20 % vol/vol du volume total de gaz.

**2.** Mousse sclérosante selon la revendication 1, **caractérisée en ce que** la micromousse comprend de l'hélium en une quantité de 1 % à 10 % du volume total de gaz.

**3.** Mousse sclérosante selon la revendication 2, **caractérisée en ce que** la micromousse comprend de l'hélium en une quantité de 1 % à 5 % du volume total de gaz.

**4.** Mousse sclérosante selon la revendication 3, **caractérisée en ce que** le mélange de gaz comprend au moins 99 % d'oxygène ou de dioxyde de carbone.

**5.** Mousse sclérosante selon l'une quelconque des revendications précédentes, **caractérisée en ce que** le liquide sclérosant est une solution de polidocanol ou de tétradécylsulfate de sodium dans un véhicule aqueux.

**6.** Mousse sclérosante selon la revendication 5, **caractérisée en ce que** le liquide sclérosant est une solution de 0,25 à 5 % vol/vol de polidocanol.

**7.** Mousse sclérosante selon l'une quelconque des revendications précédentes, **caractérisée en ce que** la micromousse est telle que moins de 20 % des bulles constituant la micromousse font moins de 30 $\mu$m de diamètre, plus de 75 % font entre 30 et 280 $\mu$m de diamètre, moins de 5 % font entre 281 et 500 $\mu$m diamètre, et il n'y a substantiellement pas de bulles de plus de 500 $\mu$m de diamètre.

**8.** Mousse sclérosante selon l'une quelconque des revendications précédentes, **caractérisée en ce que** le rapport gaz/liquide dans le mélange est contrôlé de sorte que la masse volumique de la micromousse soit de 0,07 g/ml à 0,19 g/ml.

**9.** Mousse sclérosante selon l'une quelconque des revendications précédentes, **caractérisée en ce que** la micromousse a une demi-vie d'au moins 2 minutes.

**10.** Procédé pour produire une micromousse adaptée pour utilisation dans une scléropathie des vaisseaux sanguins, comprenant l'introduction d'un gaz dispersible dans le sang physiologiquement acceptable dans un récipient contenant un liquide sclérosant aqueux et la libération du mélange de gaz dispersible dans le sang et de liquide sclérosant, de telle manière qu'après la libération du mélange, les composants du mélange interagissent de manière à former une micromousse, **caractérisé en ce que** le gaz dispersible dans le sang physiologiquement acceptable comprend 75 % vol/vol ou plus d'oxygène et/ou de dioxyde de carbone et moins de 10 % vol/vol d'azote qui est stocké en présence d'hélium en une quantité de 0,5 % à 20 % du volume total de gaz.

**11.** Procédé selon la revendication 10, **caractérisé en ce que** le composant d'oxygène du mélange de gaz final est stocké dans un récipient séparé du liquide sclérosant aqueux et introduit immédiatement avant utilisation.

**12.** Dispositif pour produire une micromousse adaptée pour utilisation dans une scléropathie des vaisseaux sanguins, comprenant un boîtier dans lequel est situé une chambre pressurisable contenant une solution de l'agent sclérosant dans un solvant physiologiquement acceptable ; un chemin avec un ou plusieurs orifices de sortie par desquels la Solution peut passer de la chambre pressurisable vers l'extérieur du dispositif par l'intermédiaire desdits un ou plusieurs orifices de sortie et un mécanisme par lequel le chemin de la chambre vers l'extérieur peut être ouvert ou fermé de sorte que, lorsque le récipient est pressurisé et le chemin est ouvert, le fluide est poussé le long du chemin et à travers les un ou plusieurs orifices de sortie ;

ledit boîtier incorporant une entrée pour l'admission d'une source pressurisée de gaz physiologiquement acceptable qui est dispersible dans le sang ; le gaz étant en contact avec la solution après l'activation du mécanisme de manière à produire un mélange de gaz-solution ;

ledit chemin vers l'extérieur du boîtier comprenant un ou plusieurs éléments moussants

**caractérisé en ce que** le boîtier est chargé avec un gaz comprenant 75 % vol/vol ou plus d'oxygène et/ou de dioxyde de carbone et moins de 10 % vol/vol d'azote qui est stocké en présence d'hélium en une quantité de 0,5 % à 20 % du volume total de gaz.

**13.** Mousse sclérosante selon l'une quelconque des revendications 1 à 10 pour sclérothérapie.

# Fig. 1

Fig. 2

EP 1 597 171 B1

Vacuum

Mass
Spec.

He Detection

Fig. 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0656203 A
- US 5676962 A
- WO 0072821 A1
- WO 0241872 A1

**Non-patent literature cited in the description**

- **KIRSCH et al.** *J Pharm Sci Tech,* 1997, vol. 51 (5), 187-194
- Foam Sclerotherapy: State of the Art. 45-50